# EUROPEAN PATENT SPECIFICATION

(11) **EP 0 891 323 B1**
(45) Date of publication and mention of the grant of the patent: **14.06.2006**
(21) Application number: 97917016.4
(22) Date of filing: 27.03.1997
(51) Int. Cl.: C07C 253/10

(54) **CATALYZED VAPOR-PHASE HYDROCYANATION OF DIOLEFINIC COMPOUNDS**
KATALYSIERTE GASPHASEN-ADDITION VON CYANWASSERSTOFF AN DIOLEFINE
HYDROCYANATION DE COMPOSES DIOLEFINIQUES PAR CATALYSE EN PHASE VAPEUR

(30) Priority: 02.04.1996 US 14618 P
(43) Date of publication of application: 20.01.1999
(60) Divisional application: 05077325.8
(73) Proprietor: INVISTA Technologies S.à.r.l., Wilmington, DE 19808 (US)
(72) Inventor: DRULINER, Joe, Douglas, Newark, DE 19711 (US); HERRON, Norman, Newark, DE 19711 (US)
(74) Representative: Carpmaels & Ransford
(86) International application number: PCT/US1997/004903
(87) International publication number: WO 1997/036855

(56) References cited:
- EP-A- 0 553 815
- US-A- 3 978 000
- US-A- 4 756 898
- US-A- 4 832 881
- US-A- 5 261 948
- US-A- 5 300 272
- US-A- 5 449 807

## Description

### FIELD OF THE INVENTION

This invention generally relates to a vapor-phase process for the hydrocyanation of diolefinic compounds to produce nonconjugated acyclic nitriles utilizing zero-valent nickel and a multidentate phosphite ligand as catalysts.

### BACKGROUND OF THE INVENTION

Catalytic hydrocyanation systems, particularly pertaining to the hydrocyanation of olefins, are known in the art. For example, liquid-phase systems useful for the hydrocyanation of butadiene to form pentenenitriles (PN) are known in the art, e.g., U.S. Patent 3,766,237. As used in that patent, and as will be used herein, the term "pentenenitrile" is intended to mean a cyanobutene. Likewise, "butenenitrile" is intended to mean a cyanopropene. The pentenenitriles so formed are further subjected to hydrocyanation and, in some cases isomerization, to form adiponitrile (ADN), a commercially important material in the manufacture of nylon. Multidentate phosphite ligands complexed to zero-valent nickel and platinum are known to be useful in the liquid-phase hydrocyanation of butadiene, as described by Baker et al., J. Chem. Soc. Chem. Commun., 1991, pages 803.804.

The overwhelming majority of prior art processes for the hydrocyanation of butadiene are conducted in the liquid phase, with all attendant waste disposal problems. Most of the previous approaches toward carrying out vapor-phase hydrocyanation of olefinic compounds have usually started with monoolefinic, not diolefinic, compounds and have given rise primarily to saturated products, which could not be further hydrocyanated. U.S. Patent 5,449,807 describes a catalyzed vapor-phase process for the hydrocyanation of diolefinic compounds comprising, reacting an acylic aliphatic diolefinic compound, preferably butadiene, with HCN in the vapor phase within a temperature range of from about 135°C to about 170°C in the presence of a supported catalyst composition comprising zero-valent nickel and at least one selected bidentate phosphite. Typically, in accordance with U.S. Patent 5,449,807, the zero-valent nickel catalysts are dispersed on silica, alumina or carbon supports in a conventional manner.

While the vapor-phase hydrocyanation process described in U.S. Patent 5,449,807 offers many advantages over liquid-phase process for optimum commercial use, the life of the catalyst needs to be prolonged. Therefore, what is needed is a process which provides for longer catalyst life in the vapor-phase hydrocyanation by the selection of catalysts, catalyst supports and catalyst support pre-treatments. Other objects and advantages of the invention will become apparent to those skilled in the art upon reference to the detailed description which hereinafter follows.

### SUMMARY OF THE INVENTION

The present invention provides a process for the vapor-phase hydrocyanation of diolefinic compounds comprising, reacting an acyclic aliphatic diolefinic compound, preferably butadiene, with HCN in the vapor phase within a temperature range of from about 135°C to about 300°C in the presence of a supported catalyst composition comprising zero-valent nickel and at least one multidentate phosphite ligand selected from the group consisting of compounds represented by Formula II: 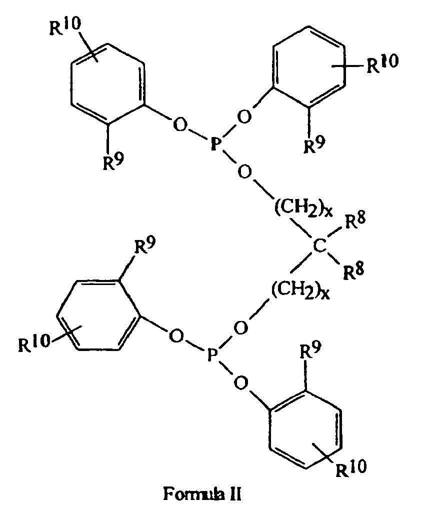 wherein
each R⁸ is, independently, H, a primary, secondary or tertiary hydrocarbyl of 1 to 12 carbon atoms, or B, where B is a substituent of the formula 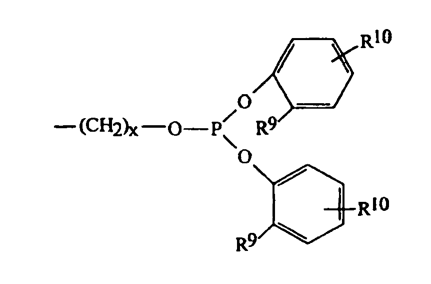 wherein x is an integer from 1 to 12;
and
each R⁹ and R¹⁰ are independently, H, OR¹¹ wherein R¹¹ is a C₁ to C₁₂ alkyl or a primary, secondary or tertiary hydrocarbyl of 3 to 12 carbon atoms and wherein R¹⁰ can be ortho, meta or para to the oxygen; and wherein x is an integer from 1 to 12;
to produce acylic pentenenitriles in which the olefinic double bond is not conjugated with the cyano group.

The process may be carried out continuously or batchwise.

In the above definitions for Formula II, the terms "secondary" and "tertiary" refer to the carbon atom attached to the aromatic ring. In addition, for purposes of the present disclosure and claims, the terms "alkenenitrile", "pentenenitrile", and "butenenitrile" are intended to mean, respectively, a cyanoalkene in which the carbon atom of the cyano group is the first carbon; a cyanobutene; and a cyanopropene.

When 1,3-butadiene (BD) is utilized as the starting material, the inventive vapor-phase hydrocyanation process produces a mixture of pentenenitriles (PN) consisting essentially of 3-pentenenitrile (3PN), 4-pentenenitrile (4PN) and 2-methyl-3-butenenitrile (2M3BN). Small amounts of dinitriles (DN) and of a dimer of 1,3-butadiene, 2-vinylcyclohexene (VCH) are also sometimes produced.

### DETAILED DESCRIPTION OF THE PREFERRED EMBODIMENTS

Catalysts useful in the practice of the present invention consist essentially of the multidentate phosphite complexes detailed above. When the catalysts are selected from those of Formula 11, they can be supported on conventional carriers of silica, alumina, carbon, or other suitable supports, as well as the specialized supports discussed hereinbelow. Commonly used techniques for treatment of supports with metal catalysts can be found in B. C. Gates, Heterogeneous Catalysis, Vol. 2, pp. 1-29, Ed. B. L. Shapiro, Texas A & M University Press, College Station, Texas, 1984. Alternately, a given support can be prepared with a uniformly dispersed coating of zero-valent nickel metal and then can be treated with a multidentate phosphite ligand. Typically, in accordance with this invention, the zero-valent nickel catalysts are dispersed on the chosen support at concentrations sufficient to produce a supported catalyst composition containing 0.3% wt. to 1.0% wt. Ni.

For prolonged catalyst life, when the catalysts are selected from those of Formula II, they should be supported on catalyst supports selected from heat-treated carbon or organic polymeric supports. The carbon utilized can be any form of commercially available particulate carbon, but the carbon must be heated in an inert atmosphere to at least 600°C, and preferably to at least 800°C, for at least 2 hours, and preferably at least 4 hours, prior to contact with the catalyst and subsequent use. After the carbon has been heat-treated and cooled, the catalyst can be applied thereto by dispersion of the catalyst in an organic liquid, such as toluene or tetrahydrofuran, and contact of this dispersion with the heat-treated carbon support. The organic polymeric supports of choice are those with thermal stabilities greater than 200°C, for example, polydivinylbenzene/polyacrylonitrile and cross-linked polystyrene and copolymers or blends thereof. In the case of organic polymeric supports, the catalyst of choice can be dispersed therewith in the same manner as utilized for heat-treated carbon supports.

The catalysts are then loaded into tubular reactors, and a gaseous diolefinic compound, e.g., butadiene, and HCN is passed continuously over the solid catalysts at temperatures sufficiently high to maintain the starting materials as well as the reaction products in the vapor phase. The temperature range is generally from about 135°C to about 300°C and preferably from about 145°C to 200°C. The temperature must be high enough to maintain all the reactants and products in the vapor phase but low enough to prevent deterioration of the catalyst. The particular preferred temperature depends to some extent on the catalyst being used, the diolefinic compound being used, and the desired reaction rate. The operating pressure is not particularly critical and can conveniently be from about 1-10 atmospheres (101.3 to 1013 kPa). No practical benefit is obtained when operating above the upper limit of this pressure range.

HCN and/or diolefinic compound starting materials can be delivered as a neat vapor or as a preheated solution in a solvent, such as acetonitrile or toluene. Under atmospheric pressure, using nitrogen or another inert gas as a carrier, temperatures of from about 140-160°C are typically used. Nitrogen is preferred because of its low cost. Gaseous oxygen, water vapor, or other gaseous substance which could react with the HCN, the zero-valent nickel catalyst, or the starting diolefinic compound should be avoided. The reaction products are liquid at room temperature and are conveniently recovered by cooling. Branched 2-methyl-3-butenenitrile can be separated from linear 3-pentenenitrile and 4-pentenenitrile by distillation.

Zero-valent nickel is known to the art and can be made in a number of ways. Most common zero-valent nickel species, which can be used to form the catalytic compositions useful in the present invention, are derived from Ni(O) complexes containing o-tritolylphosphite, p-tritolylphosphite, cyclooctadiene, and ethylene. Ni(O) can also be prepared by reduction of Ni(II) compounds with molecular hydrogen, or other reducing agents, in the presence of appropriate ligands (e.g., where Ni(NO₃)₂ is reduced by H₂ to provide Ni(O) on silica gel). Moreover, Ni(O) complexes containing multidentate ligands can be prepared from reduction of Ni(II) compounds (e.g., where Ni(ligand "A")(ethylene) is prepared) and Ni metal and a multidentate ligand. Other zero-valent nickel species known to those skilled in the art can be successfully used as well.

The actual catalyst is a complex of zero-valent nickel with the multidentate ligand, which is formed when those two materials are combined. An effective catalyst requires at least one mole of multidentate ligand for one gram-atom of zero-valent nickel.

The diolefinic compound reactants used in this invention include primarily conjugated diolefins containing from 4 to 10 carbon atoms; for example 1,3-butadiene and cis and trans-2,4-hexadienes. Butadiene is especially preferred by reason of its commercial importance in the production of adiponitrile. Other suitable diolefinic compounds include diolefinic compounds substituted with groups which do not deactivate the catalyst, for example, cis and trans-1,3-pentadienes.

The following Formulas III and IV illustrate suitable representative starting diolefinic compounds; and Formulas V, VI, and VII represent the products obtained from 1,3-butadiene and HCN.

CH₂=CH-CH=CH₂ (1,3-butadiene) III

R¹²CH=CH-CH=CHR¹³ IV

wherein each one of R¹² and R¹³, independently, is H or a C₁ to C₃ alkyl.

NC-CH₂-CH=CH-CH₃ V (3PN)

CH₂=CH-CH₂-CH₂-CN VI (4PN)

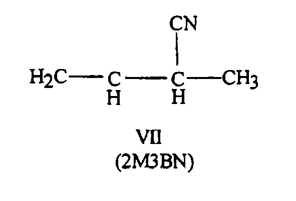

It will be recognized that Compound III is a special case of Formula IV, where each one of R¹² and R¹³ is hydrogen.

In the practice of the hydrocyanation process of the present invention, a reactor, such as a tubular reactor, is charged in an inert atmosphere with the desired supported Ni(O) (Ligand) catalyst. The reactor feed and exit lines are preferably purged with an inert gas, such as N₂, Ar, or He. The reactor is then heated to the desired temperature, either under a continuous flow of inert gas or sealed from the ambient atmosphere. The reactor is fed with the desired diolefinic compound and HCN. These may be fed together or separately, either neat or as solutions in suitable solvents, such as acetonitrile or toluene. When the reactants are fed continuously, an inert gas carrier normally is employed as well. The diolefinic compound, HCN and any solvent are passed through a heated portion of feed line heated to the reaction temperature to ensure complete vaporization. The gaseous product mixture exiting the reactor can be passed, if desired, through a heated gas sampling loop of a gas chromatograph for perodically monitoring the progress of the reaction. Alternatively, the gaseous effluent can be cooled to about 0°C to 25°C in order to condense all products to liquids. The flow rate of the diolefinic compound preferably is such that its mole ratio to catalyst, per hour of continuous feed, is about 5:1 to 100:1. The mole ratio of the diolefinic compound to HCN normally is at least about 1:1.

The hydrocyanation reaction is preferably carried out without a solvent. If any solvent is used, the solvent should be gaseous at the reaction temperature and pressure and inert towards the diolefinic compound, HCN, and the catalyst. Such solvents include hydrocarbons such as hexane, benzene, toluene or xylene, or nitriles such as acetonitrile.

### EXAMPLES

This invention is now illustrated by the following non-limiting examples of certain preferred embodiments thereof, wherein all parts, proportions, and percentages are by weight, unless otherwise indicated. In the Examples, Ligand "PE-1" is the ligand of Formula II, where x is 1 and each R¹⁰ is CH₃, each R⁹ is isopropyl and each R⁸ is B.

### EXAMPLE 1

### Synthesis of Silica-supported

Ni[OTTP]₂CH₂=CH₂ + PE-1

where OTTP is o-tritolyl phosphite and the

Ligand "PE-1" is the ligand of Formula II,
where x =1 and each R⁸ is B, each R⁹ is isopropyl and each R¹⁰ is methyl

A 2 g sample of Johnson Mathey Co. silica (300 m²/g, 8/12 mesh) was dried under flowing N₂ at 200°C for 2 hours, then cooled to ambient temperature in a N₂-filled glove box. The 2 g of dried silica was combined with 0.21 g (0.125 mmole) Ligand PE-1, 0.10 g (0.125 mmole) Ni(OTTP)₂CH₂=CH₂ and 10 mL dry tetrahydrofuran (THF) and the mixture was stirred for 15 minutes. Solvent THF was evaporated under vacuum to provide the silica-supported Ni(0) catalyst used for Example 5 in Table 1.

### EXAMPLE 2

### Vapor-Phase Hydrocyanation of Butadiene (BD)

An empty 0.25-inch (0.64 cm) diameter, 15-inch (37.5 cm) long stainless steel tubular reactor was placed in a nitrogen-filled glove box. A plug of glass wool was placed in the bottom end of the reactor, followed by the amount and type of Ni(O) catalyst shown in Table 1. A thermocouple was inserted into the top of the reactor. Both ends of the reactor were sealed with metal fittings, and the reactor was removed from the glove box and connected to stainless steel reactor feed lines purged with nitrogen. The feed streams of nitrogen, butadiene (BD), HCN and toluene solvent were preheated to 145°C to ensure complete vaporization. The reactor was heated in a split tube furnace to the temperatures shown in Table 1. Gaseous effluent from the reactor passed through a heated sampling valve which permitted periodic on-line gas chromatographic analyses of products. Feed solutions of weighed amounts of butadiene in toluene also contained weighed amounts of acetonitrile which served as an internal gas chromatograph (GC) standard. GC analyses were done on a 30 m DB-23 capillary columnn of a 0.32 mm internal diameter, supplied by J&W Scientific, Folsom, California. The stationary phase was cyanopropyl (50%) methylpolysiloxane. Table 1 shows the specific reaction conditions and summarizes the results.

**TABLE 1 Vapor Phase BD/HCN Runs**

| Ex No. | Catalyst (Support) | Catalyst g, % Ni | Feed mmole/hr | | Feed mL/min N₂ | Temp. °C | Elapsed Time (Hr.) | % Conv. to PN's | % Selectivity 3PN, 4PN, 2M3BN | % Conv. to DN's |
|---|---|---|---|---|---|---|---|---|---|---|
| | | | BD | HCN | | | | | | |
| 2 | Ni(OnP)₂CH₂=CH₂ + L | 1.02,0.31 | .51 | 0.26 | 10 | 145 | 1 | 86.5 | 95.9 | |
| | L=Ligand "PE- 1" | | | | | | 5 | 96.7 | 96.9 | |
| | (Silica) | | | | | | 11 | 95.8 | 96.5 | 4.1 |
| | Example 1 | | | | | | 20 | 98.7 | 95.8 | 3.8 |

In the above Table, percent conversion to pentenenitriles (PNs) was calculated as (measured GC area % for PNs/GC area % for acetonitrile) x (GC response factor for PNs) x (g of acetonitrile fed per hour/mmoles of BD fed per hour) x 100. Response factor is the number, characteristic for each compound, required for converting area percent to weight percent/molecular weight of PNs.

Percent conversion to DNs was determined as (measured GC area % for DNs)/(GC area % for acetonitrile) x (GC response factor for DNs) x (g of acetonitrile fed per hour./mmoles of BD fed per hour) x 100.

Percent selectivity to (3PN + 4PN + 2M3BN) was determined as (GC area % for 3PN + 4PN + 2M3BN)/(GC area % for PNs + DNs) x 100.

## Claims

1. A process for the vapor-phase hydrocyanation of diolefinic compounds comprising, reacting an acyclic aliphatic diolefinic compound with HCN in the vapor phase within a temperature range of from 135°C to 300°C in the presence of a supported catalyst composition comprising zero-valent nickel and at least one multidentate phosphite ligand represented by Formula II: 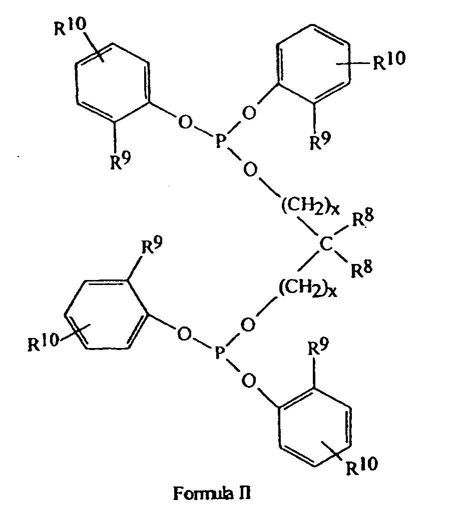 wherein
each R⁸ is, independently, H, a primary, secondary or tertiary hydrocarbyl of 1 to 12 carbon atoms, or B, where B is a substituent of the formula 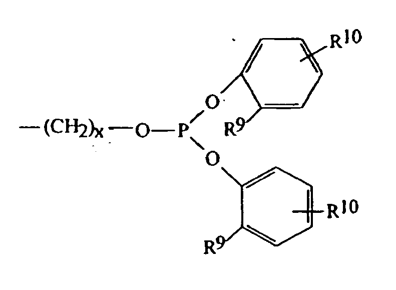 wherein x is an integer from 1 to 12; and
each R⁹ and R¹⁰ are independently, H, OR¹¹ wherein R¹¹ is a C₁ to C₁₂ alkyl or a primary, secondary or tertiary hydrocarbyl of 3 to 12 carbon atoms and wherein R¹⁰ can be ortho, meta or para to the oxygen; and
wherein x is an integer from 1 to 12;
to produce acyclic pentenenitriles in which the olefinic double bond is not conjugated with the cyano group.

2. The process of claim 1 wherein the catalyst support is selected from the group consisting of silica, alumina and carbon.

3. The process of claim 1 wherein the acyclic aliphatic diolefinic compound is selected from the group consisting of 1,3-butadiene, cis-2,4-hexadiene, and trans-2,4-hexadiene.

4. The process of claim 1 wherein the temperature range is from 145°C to 200°C.

5. The process of claim 1 wherein the acyclic pentenenitriles produced include 3-pentenenitrile and 4-pentenenitrile.

## Patentansprüche

1. Verfahren zur Dampfphasen-Hydrocyanierung von Diolefinverbindungen, aufweisend, Reaktion einer acyclischen aliphatischen Diolefinverbindung mit HCN in der Dampfphase innerhalb eines Temperaturbereichs von 135°C bis 300°C in Gegenwart einer Trägerkatalysatorzusammensetzung, die nullwertiges Nickel und mindestens einen mehrzähnigen Phosphitliganden aufweist, der durch Formel II dargestellt wird: 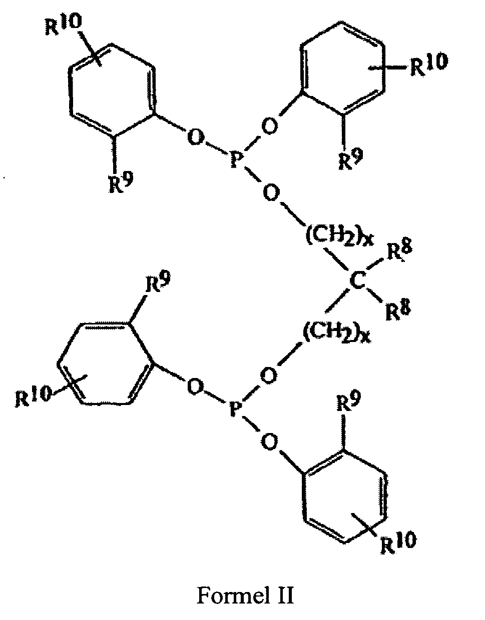 wobei
jedes R⁸ unabhängig voneinander H, ein primäres, sekundäres oder tertiäres Hydrocarbyl mit 1 bis 12 Kohlenstoffatomen oder B ist, wobei B ein Substituent der Formel 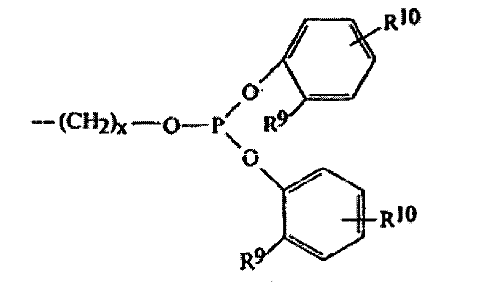 ist, wobei x eine ganze Zahl von 1 bis 12 ist; und
jedes R⁹ und R¹⁰ unabhängig voneinander H, OR¹¹, wobei R¹¹ ein C₁- bis C₁₂₋Alkyl oder ein primäres, sekundäres oder tertiäres Hydrocarbyl mit 3 bis 12 Kohlenstoffatomen ist, und wobei R¹⁰ ortho-, meta- oder paraständig zu dem Sauerstoff sein kann; und
wobei x eine ganze Zahl von 1 bis 12 ist;
um acyclische Pentennitrile herzustellen, in denen die Olefindoppelbindung nicht mit der Cyangruppe konjugiert ist.

2. Verfahren nach Anspruch 1, wobei der Katalysatorträger aus der Gruppe ausgewählt ist, die aus Siliciumoxid, Aluminiumoxid und Kohlenstoff besteht.

3. Verfahren nach Anspruch 1, wobei die acyclische aliphatische Diolefinverbindung aus der Gruppe ausgewählt ist, die aus 1,3-Butadien, cis-2,4-Hexadien und trans-2,4-Hexadien besteht.

4. Verfahren nach Anspruch 1, wobei der Temperaturbereich von 145°C bis 200°C reicht.

5. Verfahren nach Anspruch 1, wobei zu den erzeugten acyclischen Pentennitrilen 3-Pentennitril und 4-Pentennitril gehören.

## Revendications

1. Procédé d'hydrocyanuration en phase vapeur de composés dioléfiniques comprenant, la réaction d'un composé dioléfinique aliphatique acyclique avec HCN dans la phase vapeur à l'intérieur d'une plage de températures allant de 135°C à 300°C en présence d'une composition de catalyseur supportée comprenant du nickel de valence nulle et au moins un ligand phosphite multidenté représentée par la formule II : 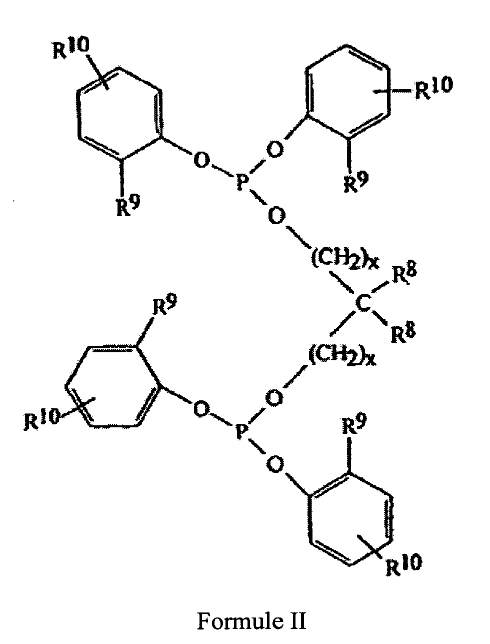 dans laquelle
chaque R⁸ représente, indépendamment, H, un hydrocarbyle primaire, secondaire ou tertiaire de 1 à 12 atome(s) de carbone, ou B, où B représente un substituant de formule 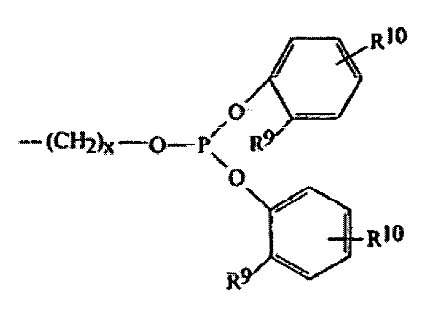 dans laquelle x représente un nombre entier ayant une valeur allant de 1 à 12 ; et
chaque R⁹ et R¹⁰ représentent indépendamment H, OR¹¹ dans laquelle R¹¹ représente un alkyle en C₁ à C₁₂ ou un hydrocarbyle primaire, secondaire ou tertiaire de 3 à 12 atomes de carbone et dans laquelle R¹⁰ peut être en position *ortho, méta ou para* par rapport à l'oxygène ; et
dans laquelle x représente un nombre entier ayant une valeur allant de 1 à 12 ;
pour produire des pentènenitriles acycliques dans lesquels la double liaison oléfinique n'est pas conjuguée au groupe cyano.

2. Procédé selon la revendication 1, dans lequel le support catalyseur est choisi parmi le groupe constitué de la silice, de l'alumine et du carbone.

3. Procédé selon la revendication 1, dans lequel le composé dioléfinique aliphatique acyclique est choisi parmi le groupe constitué du 1,3-butadiène, *cis*-2,4-hexadiène, et *trans*-2,4-hexadiène.

4. Procédé selon la revendication 1, dans lequel la plage de températures se situe de 145°C à 200°C.

5. Procédé selon la revendication 1, dans lequel les pentènenitriles acycliques produits incluent le 3-pentènenitrile et 4-pentènenitrile.
